# EUROPEAN PATENT APPLICATION

(11) **EP 4 792 802 A1**
(43) Date of publication of application: **19.08.2026**
(21) Application number: 26158085.6
(22) Date of filing: 12.02.2026
(51) Int. Cl.: A61K 8/73, A61K 8/92, A61Q 17/04

(54) **SUNSCREEN COMPOSITIONS**

(30) Priority: 13.02.2025 EP 25157801; 04.06.2025 DK PA202570077
(71) Applicant: Persano Group A/S, 3230 Græsted (DK)
(72) Inventor: DEGN, Jannie, 3230 Græsted (DK)
(74) Representative: Aera A/S

(57) **Abstract**

This invention relates to compositions that are used in the cosmetic industry, specifically sunscreen compositions. The compositions are water resistant with active sunscreen agents, and does not comprise microplastics.

## Description

### FIELD

This invention relates to compositions that are used in the cosmetic industry, specifically sunscreen compositions. The compositions are water resistant with active sunscreen agents, and does not comprise microplastics, e.g. synthetic polymer microparticles (SPM).

### BACKGROUND

Sunscreen compositions applied to the skin provide protection against the damaging effects of ultraviolet radiation. The general knowledge regarding the damaging effects to skin from the sun's ultraviolet radiation has grown. As a result, use of sunscreen compositions has become the norm for people who spend time in sunlight.

Sunscreens are a complex mixture of active and inactive ingredients, and can be found in various forms (e.g., lotions, sprays, roll-ons). The effectiveness of sunscreen is determined by the combination of UV filters and inactive ingredients. Important considerations for formulations such as SPF (sun protection factor), broad-spectrum coverage, photostability, substantivity, and cosmetic appeal are key for the identification of optimal sunscreen compositions.

Sunscreen compositions include at least one active sunscreen agent, which is typically an ultraviolet light-absorbing ingredient. Because active sunscreen agents generally absorb ultraviolet light in a limited range of wavelengths, sunscreen compositions often utilize multiple active sunscreen agents which offer absorption of different or partially overlapping ranges of wavelengths.

In order to effectively offer protection from ultraviolet radiation, sunscreen agents must have good substantivity, i.e., a tendency to resist being easily removed or to persist on the skin. For example, it is important that a sunscreen composition be resistant to removal via normal rubbing or brushing against clothes, furniture, or other normal contact experienced by a person's skin.

Many consumers use sunscreen compositions in connection with water sports, on hot days, and/or in connection with strenuous physical activities. It is therefore important that a sunscreen composition have water resistance so that it is not lost due to contact with water or perspiration.

A challenge for the cosmetic industry working with sunscreen compositions relates to the use of polymers such as microplastics (e.g. synthetic polymer microparticles (SPMs)) for the use in such compositions. These polymers are used for various purposes including to bind together the product ingredients so that the cream does not split or separate in the tube, and for protection because the use of helps to add waterproof properties to your sunscreen, giving you added sun protection in the water, or when you are doing sweat-inducing activities in the sun.

Plastics make our lives easier in many ways and are often lighter or cost less than alternative materials. However, if they are not properly disposed of or recycled, they may end up in the environment where they stay for centuries and degrade into smaller and smaller pieces. These small pieces (typically smaller than 5mm) are called microplastics and they are of concern.

Microplastics are solid plastic particles composed of mixtures of polymers and functional additives. They may also contain residual impurities. Microplastics are also manufactured and added to products for specific purposes, and in sunscreen for several reasons.

While the cosmetic industry continues its ongoing effort to identify a method of forming a sufficiently water-resistant sunscreen that meets the consumers' other requirements, there remains a need for sunscreen compositions having improved substantivity, especially in terms of water resistance and to limit of completely restrict the use of synthetic polymers and microplastics.

The challenge related to polymers and microplastics in sunscreen compositions is becoming an increasing problem in the cosmetic industry due to environmental considerations, health concerns etc.

Accordingly, it is desirable to provide a sunscreen composition for topical application having improved parameters.

Furthermore, other desirable features and characteristics of the present invention will become apparent from the subsequent detailed description of the invention and the appended claims, taken in conjunction with this background of the invention.
US 2022/0354772 A1 (CLAUS, J. et al.) discloses a composition that comprises at least one organic water insoluble thermoplastic polymer
US 2025/0041179 A1 (GENRICH, F. et al.) discloses one water resistance agent.

The two documents do not disclose a specific combination that solves the technical challenge of providing a sunscreen composition that retain the effective UV filter and water resistance while not comprising microplastic polymers.

### SUMMARY

The present invention relates to a cosmetic composition comprising at least one sunscreen agent, at least one water resistance agent, and at least one organic water insoluble thermoplastic polymer.

In one or more embodiments of the present invention, the cosmetic composition is a sunscreen composition.

In one or more embodiments of the present invention, the water resistance agent is selected from the group consisting of hydrolysed jojoba ester, Hydrogenated Jojoba Oil, Hydrogenated Olive Oil Stearyl Ester, Hydrogenated Olive Oil Unsaponifiables, Hydrogenated Palm Glycerides Citrate, Hydrogenated Palm Oil, Hydrogenated Polydecene, Shea Butter Ethyl Esters, Hydrogenated Castor Oil, Hydrogenated Vegetable Oil, Hydrogenated Polyisobutene, Hydrogenated Rosin, Shorea Robusta Resin, Lanolin Wax and Candelilla Cera.

In one or more embodiments of the present invention, the water resistance agent is hydrolysed jojoba ester.

In one or more embodiments of the present invention, the water resistance agent is hydrolysed jojoba ester, and wherein the amount of hydrolysed jojoba ester in the composition is 0,1-1%.

In one or more embodiments of the present invention, the organic water insoluble thermoplastic polymer is a polysaccharide thickening agent selected from the group consisting of ethylcellulose, cellulose, carboxymethyl hydroxyethylcellulose, hydroxypropyl methylcellulose, methyl hydroxyethylcellulose, microcrystalline cellulose, sodium cellulose sulphate, hydroxyethylcellulose, hydroxypropylcellulose, and any mixtures thereof.

In one or more embodiments of the present invention, the organic water insoluble thermoplastic polymer is ethylcellulose.

In one or more embodiments of the present invention, the organic water insoluble thermoplastic polymer is ethylcellulose, and wherein the amount of ethylcellulose in the composition is 0,01-0,5%.

In one or more embodiments of the present invention, the composition is comprising at least one sunscreen agent, hydrolysed jojoba ester, and ethylcellulose.

In one or more embodiments of the present invention, the composition is comprising at least one sunscreen agent, 0,1-1% hydrolysed jojoba ester, and 0,01-0,5% ethylcellulose.

In one or more embodiments of the present invention, the composition is further comprising jojoba esters.

In one or more embodiments of the present invention, the composition is further comprising water.

In one or more embodiments of the present invention, the composition is further comprising glycerin.

In one or more embodiments of the present invention, the at least one sunscreen active is selected from the group consisting of Cinoxate, Polysilicone-15, Dioxybenzone, Homosalate, Menthyl anthranilate, Octocrylene, Padimate O, Phenylbenzimidazole sulfonic acid, Titanium dioxide, Trolamine salicylate, Zinc oxide, Benzophenone-3 , Ethylhexyl Methoxycinnamate, Butyl Methoxydibenzoylmethane (BMBM), Diethylamino hydroxybenzoyl Hexyl Benzoate, Diethylhexyl Butamido Triazone, PABA, camphor benzalkonium methosulfate, terephthalidene dicamphor sulfonic acid, benzylidene camphor sulfonic acid, polyacrylamidomethyl benzylidene camphor, PEG-25 PABA, isoamyl p-methoxycinnamate, ethylhexyl triazone, drometrizole trielloxane, 4-methylbenzylidene camphor, 3-benzylidene camphor, ethylhexyl salicylate, ethylhexyl dimethyl PABA, benzophenone-4, methylene bis-benztriazolyl tetramethylbutylphenol, disodium phenyl dibenzimidazole tetrasulfonate, bis-ethylhexyloxyphenol methoxyphenol triazine, methylene bisbenzotriazolyl tetramethylbutylphenol, bisethylhexyloxyphenol methoxyphenyl triazine and any combination thereof, or salts or derivatives of any one or more of these compounds, or any combination hereof.

In one or more embodiments of the present invention, the cosmetic composition does not comprise microplastics, such as acrylates/C10-30 alkyl acrylate crosspolymer, acrylates/C12-22 alkyl methacrylate copolymer, carbomer, dimethicone, hydroxyethyl acrylate/sodium acryloyldimethyl taurate copolymer, triacontanyl PVP, VP/Eicosene copolymer, or e.g. VP/Hexadecene copolymer.

In one or more embodiments of the present invention, the cosmetic composition has an SPF in the range of SPF15-SPF50.

In one or more embodiments of the present invention, the cosmetic composition has SPF15.

In one or more embodiments of the present invention, the cosmetic composition has SPF30.

In one or more embodiments of the present invention, the cosmetic composition has SPF50.

In one or more embodiments of the present invention, the cosmetic composition has SPF50+

In one or more embodiments of the present invention, the cosmetic composition is selected from the group consisting of a lotion, a cream, a spray, an emulsion, a serum, a stick and a gel.

### DETAILED DESCRIPTION

Commonly used polymers that can be categorized as microplastics or synthetic polymer microparticles (SPM) can be listed as on a bottle of sunscreen as e.g. acrylates/C10-30 alkyl acrylate crosspolymer, acrylates/C12-22 alkyl methacrylate copolymer, carbomer, dimethicone, hydroxyethyl acrylate/sodium acryloyldimethyl taurate copolymer, triacontanyl PVP, VP/Eicosene copolymer, or e.g. VP/Hexadecene copolymer.

"Synthetic polymer microparticles" or "SPM" are polymers that are solid and which fulfil both of the following conditions: (a) are contained in particles and constitute at least 1 % by weight of those particles; or build a continuous surface coating on particles; (b) at least 1 % by weight of the particles referred to in point (a) fulfil either of the following conditions: (i) all dimensions of the particles are equal to or less than 5 mm; (ii) the length of the particles is equal to or less than 15 mm and their length to diameter ratio is greater than 3.

The following polymers are excluded from this designation:
(a) polymers that are the result of a polymerisation process that has taken place in nature, independently of the process through which they have been extracted,
   which are not chemically modified substances; (b) polymers that are degradable;
(c) polymers that have a solubility greater than 2 g/L; (d) polymers that do not contain carbon atoms in their chemical structure.

Synthetic polymers microparticles "SPMs" that fall under this definition shall not be placed on the market as substances on their own, or in a concentration equal to or greater than 0.01 % by weight, as of October 17th, 2023.

There are exceptions and transition periods. This regulation also comes with labelling obligations and reporting requirements.

Exception means that even though a material qualifies as a microplastic, the restriction would not apply.

One example is that the restriction does not apply to the placing on the market of synthetic polymer microparticles, as substances on their own or in mixtures, for use at industrial sites. This is the so-called "4a derogation".

There are several other exceptions but only one is relevant to the cosmetic sector: the so-called "5b derogation". This "5b derogation" states that the restriction shall not apply to the placing on the market of synthetic polymer microparticles the physical properties of which are permanently modified during intended end use in such a way that the polymer no longer falls within the scope of this regulation (see above).

In practice, it means that the microparticle no longer fulfill size criteria or solid-state criteria. This provision would derogate film-forming functions of microplastics in cosmetic products since the microplastic particles cease to exist at the point of use: e.g. they 'dissolve', 'merge' or permanently 'swell' in contact with water to such an extent that they can no longer be considered to be particles as they have lost their interface or do not fulfil size dimensions criteria (too big or too small).

It is worth noticing that a permanent modification is necessary to quality for this derogation.

It is largely under the responsibility of raw material suppliers and product manufacturers to provide this information.

Thus, there is a technical challenge in the cosmetics industry to provide products that do not contain SPMs according to the above definitions.

The present application relates to cosmetic compositions that do not contain such polymers or SPMs.

The inventor of the present invention has surprisingly found that a combination of specific classes of compounds allows for the formulation of sunscreen compositions that do not contain microplastics, while retaining the important features of comprising an effective UV filter, being water resistant etc.

Specifically, hydrolysed jojoba ester and ethylcellulose leads to a sunscreen composition that does not contain microplastics, while retaining the important features of comprising an effective UV filter while being water resistant as described in Example 1.

It is therefore important that the specific technical features of hydrolysed jojoba ester and ethylcellulose are combined to arrive at the sunscreen composition that does not contain microplastics, while retaining the important features of comprising an effective UV filter while being water resistant.

Other combinations, e.g. using cellulose or microcrystalline cellulose does not lead to this effect. Such compositions are not water resistant. Thus, the applicant has interestingly identified that ethylcellulose and cellulose are not equivalents because one leads to water resistance, and the other does not.

Thus, the present invention relates to a cosmetic composition comprising at least one sunscreen agent, at least one water resistance agent, and at least one organic water insoluble thermoplastic polymer.

In one or more embodiments of the present invention, the cosmetic composition is a sunscreen composition.

In one or more embodiments of the present invention, the cosmetic composition does not contain SPMs that are defined under 4a derogation.

In one or more embodiments of the present invention, the cosmetic composition does not contain SPMs that are defined under 5b derogation.The present invention is therefore solving the technical problem of providing a cosmetic composition that does not contain and SPMs defined under 4a derogation or under 5b derogation.

In one or more embodiments of the present invention, the cosmetic composition does not contain any polymers. This is advantageous simply because such polymers could be interpreted as SPMs by the consumer.

In one or more embodiments of the present invention, the composition is further comprising jojoba esters.

In one or more embodiments of the present invention, the composition is further comprising water.

In one or more embodiments of the present invention, the composition is further comprising glycerin.

In one or more embodiments of the present invention, the cosmetic composition does not comprise microplastics, such as acrylates/C10-30 alkyl acrylate crosspolymer, acrylates/C12-22 alkyl methacrylate copolymer, carbomer, dimethicone, hydroxyethyl acrylate/sodium acryloyldimethyl taurate copolymer, triacontanyl PVP, VP/Eicosene copolymer, or e.g. VP/Hexadecene copolymer.

### Sunscreen actives (UV filters/sunscreen agents)

SPF is a measure of how much solar energy (UV radiation) is required to produce sunburn on protected skin (i.e., in the presence of sunscreen) relative to the amount of solar energy required to produce sunburn on unprotected skin. As the SPF value increases, sunburn protection increases. By definition, SPF is determined in the laboratory by comparing the amount of UV radiation required to produce minimal redness (minimal erythemal dose, MED) on sunscreen-protected skin with that on unprotected skin.

The sunscreen composition comprises one or more sunscreen agents, which provide the adsorption or blocking of the UV radiation, before it reaches the skin. The sunscreen agent may be selected from the group consisting of Avobenzone, Cinoxate, Dioxybenzone, Homosalate, Menthyl anthranilate, Octocrylene, Octyl methoxycinnamate, Octyl salicylate, Oxybenzone, Padimate O, Phenylbenzimidazole sulfonic acid, Sulisobenzone, Titanium dioxide, Trolamine salicylate, Zinc oxide, Benzophenone-3 , Ethylhexyl Methoxycinnamate, Octocrylene, Butyl Methoxydibenzoylmethane (BMBM), Diethylamino hydroxybenzoyl Hexyl Benzoate, Diethylhexyl Butamido Triazone, PABA, camphor benzalkonium methosulfate, phenylbenzimidazole sulfonic acid, terephthalidene dicamphor sulfonic acid, benzylidene camphor sulfonic acid, polyacrylamidomethyl benzylidene camphor, PEG-25 PABA, isoamyl p-methoxycinnamate, ethylhexyl triazone, drometrizole trielloxane, 4-methylbenzylidene camphor, 3-benzylidene camphor, ethylhexyl salicylate, ethylhexyl dimethyl PABA, benzophenone-4, methylene bis-benztriazolyl tetramethylbutylphenol, disodium phenyl dibenzimidazole tetrasulfonate, bis-ethylhexyloxyphenol methoxyphenol triazine, methylene bisbenzotriazolyl tetramethylbutylphenol, bisethylhexyloxyphenol methoxyphenyl triazine and any combination thereof.

In an embodiment the at least one sunscreen agent is selected from the group consisting of Homosalate, Octocrylene, Ethylhexyl Methoxycinnamate, Butyl Methoxydibenzoylmethane (BMBM), Diethylamino Hydroxybenzoyl Hexyl Benzoate, diethylhexyl butamido triazone, ethylhexyl salicylate or any combination thereof. Even more preferred is a composition comprising Homosalate, Octocrylene, Ethylhexyl Methoxycinnamate, Butyl Methoxydibenzoylmethane (BMBM), Diethylamino Hydroxybenzoyl Hexyl Benzoate and diethylhexyl butamido triazone. The amount of each individual sunscreen agent used in a given sunscreen composition is determined by the desired SPF value and regulatory limitations for the percentage (w/w) of sunscreen agent allowed in sunscreen compositions for a given jurisdiction.

To achieve broad range UV protection, it is a legal requirement to add both in EU to add both UVA, UVB and broad range UV filters. Finally, if the desired SPF is not achieved with a given set of sunscreen agents, further amounts of a different sunscreen agent may be added or preferably the SPF may be boosted by other means, such as described herein. Therefore, in preferred embodiments the sunscreen composition may comprise several sunscreen agents to achieve the desired SPF value. Thus, in a preferred embodiment the composition comprises at least 2 sunscreen agents, such as 3 sunscreen agents, e.g. 4 sunscreen agents, such as 5 sunscreen agents, e.g. 6 sunscreen agents, such as 7 sunscreen agents.

In one or more embodiments of the present invention, the at least one sunscreen active is selected from the group consisting of Cinoxate, Polysilicone-15, Dioxybenzone, Homosalate, Menthyl anthranilate, Octocrylene, Octyl methoxycinnamate, Octyl salicylate, Padimate O, Phenylbenzimidazole sulfonic acid, Titanium dioxide, Trolamine salicylate, Zinc oxide, Benzophenone-3 , Ethylhexyl Methoxycinnamate, Octocrylene, Butyl Methoxydibenzoylmethane (BMBM), Diethylamino hydroxybenzoyl Hexyl Benzoate, Diethylhexyl Butamido Triazone, PABA, camphor benzalkonium methosulfate, terephthalidene dicamphor sulfonic acid, benzylidene camphor sulfonic acid, polyacrylamidomethyl benzylidene camphor, PEG-25 PABA, isoamyl p-methoxycinnamate, ethylhexyl triazone, drometrizole trielloxane, 4-methylbenzylidene camphor, 3-benzylidene camphor, ethylhexyl salicylate, ethylhexyl dimethyl PABA, benzophenone-4, methylene bis-benztriazolyl tetramethylbutylphenol, disodium phenyl dibenzimidazole tetrasulfonate, bis-ethylhexyloxyphenol methoxyphenol triazine, methylene bisbenzotriazolyl tetramethylbutylphenol, bisethylhexyloxyphenol methoxyphenyl triazine and any combination thereof, or salts or derivatives of any one or more of these compounds, or any combination hereof.

In one or more embodiments of the present invention, the organic water insoluble thermoplastic polymer is a polysaccharide thickening agent selected from the group consisting of ethylcellulose, cellulose, carboxymethyl hydroxyethylcellulose, hydroxypropyl methylcellulose, methyl hydroxyethylcellulose, microcrystalline cellulose, sodium cellulose sulphate, hydroxyethylcellulose, hydroxypropylcellulose, and any mixtures thereof.

In one or more embodiments of the present invention, the organic water insoluble thermoplastic polymer is ethylcellulose.

### Film former - Ethylcellulose

Ethyl cellulose (or ethylcellulose), or simply EC, is a derivative of cellulose in which some of the hydroxyl groups on the repeating glucose units are converted into ethyl ether groups. The number of ethyl groups can vary depending on the manufacturer.

It is mainly used as a thin-film coating material for coating paper, vitamin and medical pills, and for thickeners in cosmetics and in industrial processes.

Food grade ethyl cellulose is one of few non-toxic films and thickeners which are not water-soluble. This property allows it to be used to safeguard ingredients from water.

Ethyl cellulose is also used as a food additive as an emulsifier (E462).

Ethyl cellulose is commonly used as a coating material for tablets and capsules, as it provides a protective barrier that prevents the active ingredients from being released too quickly in the digestive system. EC is also used as a binder, thickener, and stabilizer in a variety of food, cosmetic, and pharmaceutical products.

Ethylcellulose is a plant-derived (from cotton or wood pulp) carbohydrate polymer that's used in cosmetics for its texture-enhancing and film-forming properties. Adding ethylcellulose to skin care formulas can help create clear gels in a range of viscosities (thicknesses). It is known to work well with various glycerides and UV filters, including octinoxate and homosalate.

This ingredient is also known as ethyl ether cellulose. It is a food-grade ingredient used to help oils gel. As such, EC considered safe as used in cosmetics.

### Water Resistance Agent

Sunscreen products may be labelled as "Water Resistant" or "Very Water Resistant" when tested in accordance with both ISO16217:20201 ('Water immersion procedure for determining water resistance') and ISO18861:20202 ('Percentage of water resistance'). They should also fulfil the following statistical criteria and thresholds: 'Water Resistant' Claim products designed to provide water resistance shall be tested by two 20-minute water immersion cycles (as described in ISO16217:20201). A product may be labelled 'water resistant' if the value for the 90% lower unilateral confidence limit [mean %WRR - d] is greater than or equal to 50% (using the method described in ISO 18861:20202) and the 95% confidence interval on the mean static SPF is within ± 17% of the mean static SPF (as described in ISO 24444:20193). The active ingredients that protect from harmful UV rays can be diminished or disrupted by moisture, including sweat and natural skin oils. As a result, the ingredients lose adherence to the skin and wash off due to sweating or swimming. In contrast, sunscreens that include water-resistant ingredients continue to be effective for a verified amount of time.

In one or more embodiments of the present invention, the water resistance agent is selected from the group consisting of hydrolysed jojoba ester, Hydrogenated Jojoba Oil, Hydrogenated Olive Oil Stearyl Ester, Hydrogenated Olive Oil Unsaponifiables, Hydrogenated Palm Glycerides Citrate, Hydrogenated Palm Oil, Hydrogenated Polydecene, Shea Butter Ethyl Esters, Hydrogenated Castor Oil, Hydrogenated Vegetable Oil, Hydrogenated Polyisobutene, Hydrogenated Rosin, Shorea Robusta Resin, Lanolin Wax and Candelilla Cera..

In one or more embodiments of the present invention, the water resistance agent is hydrolysed jojoba ester.

Hydrolyzed jojoba esters are a highly viscous, tacky gel at room temperature. They are a pale straw color and have no clearly defined melting point. They are soluble in most alcohols, glycols and water in certain pH ranges. The pH is typically above 10. When applied to skin, they form a water-resistant barrier which gives desirable substantivity to some cosmetic formulations.

The topical substantivity and water-resistance of hydrolyzed jojoba esters make them well suited to hold other substances on the surface of the skin. Hydrolyzed jojoba esters can extend the moisturizing properties of traditional emulsions, and work as a refatting agent in astringents and toners. The high pH of hydrolyzed jojoba esters make them suitable as a gel neutralizer to thicken carbomer gels. Hydrolyzed jojoba esters created via a saponification reaction contain more than 12 natural long chain fatty alcohols (C16-C26).

Thus, hydrolyzed jojoba esters are a mixture of the free fatty acids, free fatty alcohols and wax esters resulting from the saponification reaction (cleaving the ester bond) of jojoba oil. These free fatty acids and free fatty alcohols are unbranched aliphatic monounsaturates with a chain length of C16 to C26.

In one or more embodiments of the present invention, the water resistance agent is hydrolysed jojoba ester, and wherein the amount of hydrolysed jojoba ester in the composition is 0,1-1%.

In one or more embodiments of the present invention, the organic water insoluble thermoplastic polymer is ethylcellulose, and wherein the amount of ethylcellulose in the composition is 0,01-0,5%.

In one or more embodiments of the present invention, the composition is comprising at least one sunscreen agent, hydrolysed jojoba ester, and ethylcellulose.

In one or more embodiments of the present invention, the composition is comprising at least one sunscreen active, 0,1-1% hydrolysed jojoba ester, and 0,01-0,5% ethylcellulose.

In an exemplary embodiment of the present invention, the composition comprises at least one sunscreen agent, 0,08% of ethylcellulose and 0,32% of hydrolyzed jojoba esters.

In an exemplary embodiment of the present invention, the composition comprises at least one sunscreen agent, 0,24% of ethylcellulose and 0,16% of hydrolyzed jojoba esters.

In one or more embodiments of the present invention, the cosmetic composition has an SPF in the range of SPF15-SPF50.

In one or more embodiments of the present invention, the cosmetic composition has SPF15.

In one or more embodiments of the present invention, the cosmetic composition has SPF30.

In one or more embodiments of the present invention, the cosmetic composition has SPF50.

In one or more embodiments of the present invention, the cosmetic composition has SPF50+.

In one or more embodiments of the present invention, the cosmetic composition is selected from the group consisting of a lotion, a cream, a spray an emulsion, a serum, a stick and a gel.

In one or more embodiments of the present invention, the cosmetic composition is a lotion.

In an exemplary embodiment of the present invention, the cosmetic composition is a lotion with SPF15, comprising at least one sunscreen agent, 0,08% of ethylcellulose and 0,32% of hydrolyzed jojoba esters.

In an exemplary embodiment of the present invention, the cosmetic composition is a lotion with SPF30, comprising at least one sunscreen agent, 0,08% of ethylcellulose and 0,32% of hydrolyzed jojoba esters.

In an exemplary embodiment of the present invention, the cosmetic composition is a lotion with SPF50, comprising at least one sunscreen agent, 0,08% of ethylcellulose and 0,32% of hydrolyzed jojoba esters.

In an exemplary embodiment of the present invention, the cosmetic composition is a spray with SPF30, comprising at least one sunscreen agent, 0,24% of ethylcellulose and 0,16% of hydrolyzed jojoba esters.

In an exemplary embodiment of the present invention, the cosmetic composition is a spray with SPF50, comprising at least one sunscreen agent, 0,24% of ethylcellulose and 0,16% of hydrolyzed jojoba esters.

### Solvent

Solvents are ingredients which are fused to dissolve and disperse other substances (raw materials) to form a homogenous and stable mixture. The choice of solvent can affect the sunscreen's texture, feel on the skin and overall performance.

In one or more embodiments of the present invention, the solvent is selected from the group consisting of aqua, dibutyl adipate, phenoxyethyl caprylate, diisopropyl adipate, Diisopropyl Sebacate, PEG-8, Dipropylene Glycol and Pentylene Glycol.

In one or more embodiments of the present invention, at least one solvent is selected from the group consisting of aqua, dibutyl adipate, phenoxyethyl caprylate and diisopropyl adipate.

In one or more embodiments of the present invention, the composition is comprising at least one sunscreen active, at least one solvent, 0,1-1% hydrolysed jojoba ester, and 0,01-0,5% ethylcellulose.

### Emollient

Emollients (mainly esters) are used to solubilize crystalline UV filters. Complete solubility of powdery filters is required for their efficiency and ensures homogeneous distribution of UV filters in the formulation. They soften and soothe the skin when applied.

In one or more embodiments of the present invention, at least one emollient is selected from the group consisting of dibutyl adipate, phenoxyethyl caprylate, diisopropyl adipate, glyceryl laurate and jojoba esters.

In one or more embodiments of the present invention, the composition is comprising at least one sunscreen agent, at least one emollient, 0,1-1% hydrolysed jojoba ester, and 0,01-0,5% ethylcellulose.

### Skin conditioning

Skin conditioning ingredients - also known as skin conditioning agents - is a term that refers to a wide range of substances that help skin look and feel smoother, both immediately and over time. Skin conditioning ingredients include natural and synthetic emollients, humectants, and occlusive agents.

In one or more embodiments of the present invention, at least one skin conditioning agent is selected from the group consisting of Dicocoyl Pentaerythrityl Distearyl Citrate, Sodium Hyaluronate.

In one or more embodiments of the present invention, the composition is comprising at least one sunscreen agent, at least one skin conditioning agent, 0,1-1% hydrolysed jojoba ester, and 0,01-0,5% ethylcellulose.

### Humectant

Humectants are substances that attract water from the air or from deeper in the skin. In pharmaceuticals and cosmetics, humectants can be used in topical dosage forms to increase the solubility of a chemical compound's active ingredients, increasing the active ingredients' ability to penetrate skin, or its activity time.

In one or more embodiments of the present invention, the humectant is glycerin (Propane-1,2,3-triol).

In one or more embodiments of the present invention, the composition is comprising at least one sunscreen agent, at least one humectant, 0,1-1% hydrolysed jojoba ester, and 0,01-0,5% ethylcellulose.

### Viscosity controlling ingredient

Viscosity controlling ingredients that help to thicken up products so that they form a nice gel, serum or moisturizer like texture. The viscosity controlling ingredient may be a viscosity controlling wax.

In one or more embodiments of the present invention, at least one viscosity controlling ingredient is selected from a group consisting of Glyceryl Stearate, Cetearyl Alcohol, Cetyl Palmitate, Jojoba Esters and Xanthan Gum.

In one or more embodiments of the present invention, the composition is comprising at least one sunscreen agent, at least one viscosity controlling ingredient, 0,1-1% hydrolysed jojoba ester, and 0,01-0,5% ethylcellulose.

### General

It should be understood that any feature and/or aspect discussed above in connections with the compounds according to the invention apply by analogy to the methods described herein.

The following figures and examples are provided below to illustrate the present invention. They are intended to be illustrative and are not to be construed as limiting in any way.

### BRIEF DESCRIPTION OF THE FIGURES

Figure 1
Guidelines for labelling of SUN Protection Factor (SPF) number and product categories are suggested by COLIPA (The European Cosmetic Toiletry and perfumery association) in Europe.

### EXAMPLES

### Example 1 - sunscreen compositions without microplastic

This project relates to the identification of sunscreen compositions without microplastics, i.e. synthetic polymer microparticles (SPM).

It is a well-established fact that UV radiation causes skin burns, loss of skin elasticity and the appearance of wrinkles, promoting premature skin ageing. Last, but not least important, UV irradiation has carcinogenic effects. For these reasons, continuous need exits for means for protecting the skin against the harmful effects of UV irradiation.

Compositions are preferred, which have a pleasant feel and are formulated as non-greasy sprays, fluids, milks/lotions, oils, creams, emulsions, serums, sticks and gels that are easily applied to the skin. It is further preferred that the composition exhibit non-adherence to sand, as users of these compositions are often in contact with sand at beaches.

In addition, the composition needs to be convenient for the user and it must contain adequate solvent to hold the necessary amount of UV sun filters and the composition needs to have high sun protection (SPF) and good water resistance.

Generally, film forming polymers or water proofing agents are added to sunscreen compositions to enhance resistance to water in vivo.

Therefore, improved sunscreen compositions which have a high SPF rating and simultaneously maintain good water resistance without using microplastics, i.e. synthetic polymer microparticles (SPM).

The above problem of providing different sunscreen compositions without using microplastics, i.e. synthetic polymer microparticles (SPM) while maintaining a high level of water resistance in vivo and high SPF rating.

This example identified the use of Ethylcellulose & Hydrolyzed Jojoba Esters in different concentrations in different sunscreen compositions of SPF and water resistance.

### UVA & UVB Test methods

Determination of UVB protection is performed after testing IN VIVO determination of the sun protection factor (SPF) + IN VIVO determination of water resistance.

Determination of the UVA protection is performed after testing IN VITRO.

The critical wavelength to be above 370 nm.

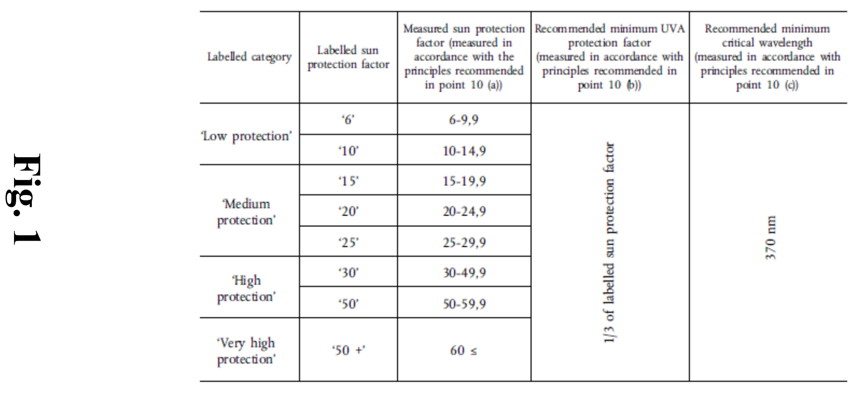

Guidelines for labelling of SUN Protection Factor (SPF) number and product categories are suggested by COLIPA (The European Cosmetic Toiletry and perfumery association) in Europe. These are listed in figure 1.

Varous sunscreen compositions have been tested. An example of a sunscreen composition on SPF 15 can be seen in Table 1.

**Table 1: Exemplary sunscreen composition SPF 15.**

| INCI/Ingredients | Active % | Function |
|---|---|---|
| Ethylcellulose | 0,08 | Filmformer |
| Hydrolyzed Jojoba Esters | 0,32 | Film former |

Ethylcellulose & Hydrolyzed Jojoba Esters were identified as key components and were therefore examined further at different SPFs.

**Table 2 is showing the amount of the concentration used of Ethylcellulose & Hydrolyzed Jojoba Esters in the different tested sunscreen compositions:**

| | SPF 15 lotion | SPF 30 lotion | SPF 50 lotion | SPF 30 spray | SPF 50 spray | SPF 50+ lotion |
|---|---|---|---|---|---|---|
| % Ethylcellulose | 0,08 | 0,08 | 0,08 | 0,24 | 0,24 | 0,1 |
| % Hydrolyzed Jojoba Esters | 0,32 | 0,32 | 0,32 | 0,16 | 0,16 | 0,48 |

**Table 3 shows the UV + WR + critical wavelength test results:**

| | SPF 15 lotion | SPF 30 lotion | SPF 50 lotion | SPF 30 spray | SPF 50 spray | SPF 50+ lotion |
|---|---|---|---|---|---|---|
| UVA | 7,8 | 12,6 | 18,9 | 14 | 16,7 | 19,7 |
| critical wavelength | 372 | 374 | 374 | 375 | 373 | 373 |
| UVB | 16,3 | 32,9 | 56,8 | 32,1 | 50,9 | 60,6 |
| Water resistance | 58,6% | 58,2% | 54,5% | 56,7% | 61,9% | 51,8% |

### Conclusion

We were able to make different sunscreen compositions with different SPF and water resistance without Microplastics = synthetic polymer microparticles (SPM). Ethylcellulose & Hydrolyzed Jojoba Esters were key components.

This example shows that Ethylcellulose & Hydrolyzed Jojoba Esters provide a sunscreen that retain water resistance and SPF without containing SPMs. Other combinations, e.g. using cellulose or microcrystalline cellulose does not lead to this effect. Such compositions are not water resistant.

### ITEMS

1. A cosmetic composition comprising at least one sunscreen agent, at least one water resistance agent, and at least one organic water insoluble thermoplastic polymer.
2. The cosmetic composition according to claim 1, which is a sunscreen composition.
3. The cosmetic composition according to claims 1-2, wherein the water resistance agent is selected from the group consisting of hydrolysed jojoba ester, Hydrogenated Jojoba Oil, Hydrogenated Olive Oil Stearyl Ester, Hydrogenated Olive Oil Unsaponifiables, Hydrogenated Palm Glycerides Citrate, Hydrogenated Palm Oil, Hydrogenated Polydecene, Shea Butter Ethyl Esters, Hydrogenated Castor Oil, Hydrogenated Vegetable Oil, Hydrogenated Polyisobutene, Hydrogenated Rosin, Shorea Robusta Resin, Lanolin Wax and Candelilla Cera, and.
4. The cosmetic composition according to claims 1-3, wherein the water resistance agent is hydrolysed jojoba ester.
5. The cosmetic composition according to claims 1-4, wherein the water resistance agent is hydrolysed jojoba ester, and wherein the amount of hydrolysed jojoba ester in the composition is 0,1-1%.
6. The cosmetic composition according to claims 1-5, wherein the organic water insoluble thermoplastic polymer is a polysaccharide thickening agent selected from the group consisting of ethylcellulose, cellulose, carboxymethyl hydroxyethylcellulose, hydroxypropyl methylcellulose, methyl hydroxyethylcellulose, microcrystalline cellulose, sodium cellulose sulphate, hydroxyethylcellulose, hydroxypropylcellulose, and any mixtures thereof.
7. The cosmetic composition according to claims 1-6, wherein the organic water insoluble thermoplastic polymer is ethylcellulose.
8. The cosmetic composition according to claims 1-7, wherein the organic water insoluble thermoplastic polymer is ethylcellulose, and wherein the amount of ethylcellulose in the composition is 0,01-0,5%.
9. The cosmetic composition according to claims 1-8, comprising at least one sunscreen agent, hydrolysed jojoba ester, and ethylcellulose.
10. The cosmetic composition according to claims 1-9, comprising at least one sunscreen agent, 0,1-1% hydrolysed jojoba ester, and 0,01-0,5% ethylcellulose.
11. The cosmetic composition according to claims 1-10, further comprising jojoba esters.
11. The cosmetic composition according to claims 1-11, further comprising water.
12. The cosmetic composition according to claims 1-12, further comprising potassium jojobate.
13. The cosmetic composition according to claims 1-13, further comprising jojoba alcohols.
14. The cosmetic composition according to claims 1-14, further comprising glycerin.
15. The cosmetic composition according to claims 1-14, wherein the at least one sunscreen active is selected from the group consisting of Cinoxate, Polysilicone-15, Dioxybenzone, Homosalate, Menthyl anthranilate, Octocrylene, Octyl salicylate, Padimate O, Titanium dioxide, Trolamine salicylate, Zinc oxide, Benzophenone-3 , Ethylhexyl Methoxycinnamate, Octocrylene, Butyl Methoxydibenzoylmethane (BMBM), Diethylamino hydroxybenzoyl Hexyl Benzoate, Diethylhexyl Butamido Triazone, PABA, camphor benzalkonium methosulfate, phenylbenzimidazole sulfonic acid, terephthalidene dicamphor sulfonic acid, benzylidene camphor sulfonic acid, polyacrylamidomethyl benzylidene camphor, PEG-25 PABA, isoamyl p-methoxycinnamate, ethylhexyl triazone, drometrizole trielloxane, 4-methylbenzylidene camphor, 3-benzylidene camphor, ethylhexyl salicylate, ethylhexyl dimethyl PABA, benzophenone-4, methylene bis-benztriazolyl tetramethylbutylphenol, disodium phenyl dibenzimidazole tetrasulfonate, bis-ethylhexyloxyphenol methoxyphenol triazine, methylene bisbenzotriazolyl tetramethylbutylphenol, bisethylhexyloxyphenol methoxyphenyl triazine and any combination thereof, or salts or derivatives of any one or more of these compounds, or any combination hereof.
16. The cosmetic composition according to claims 1-15, wherein cosmetic composition does not comprise microplastics, such as acrylates/C10-30 alkyl acrylate crosspolymer, acrylates/C12-22 alkyl methacrylate copolymer, carbomer, dimethicone, hydroxyethyl acrylate/sodium acryloyldimethyl taurate copolymer, triacontanyl PVP, VP/Eicosene copolymer, or e.g. VP/Hexadecene copolymer.
17. The cosmetic composition according to claims 1-16, wherein cosmetic composition has an SPF in the range of SPF15-SPF50+.
18. The cosmetic composition according to claims 1-17, wherein cosmetic composition has SPF15.
19. The cosmetic composition according to claims 1-17, wherein cosmetic composition has SPF30.
20. The cosmetic composition according to claims 1-17, wherein cosmetic composition has SPF50.
21. The cosmetic composition according to claims 1-20, wherein cosmetic composition is selected from the group consisting of a lotion, a cream, a spray, an emulsion, a serum, a stick and a gel.
22. The cosmetic composition according to claims 1-21, wherein cosmetic composition does not contain synthetic polymer microparticles "SPMs".
23. The cosmetic composition according to claims 1-21, wherein cosmetic composition does not contain synthetic polymer microparticles "SPMs", wherein SPM is defined as (a) are contained in particles and constitute at least 1 % by weight of those particles; or build a continuous surface coating on particles; (b) at least 1 % by weight of the particles referred to in point (a) fulfil either of the following conditions: (i) all dimensions of the particles are equal to or less than 5 mm; (ii) the length of the particles is equal to or less than 15 mm and their length to diameter ratio is greater than 3.

## Claims

1. A sunscreen composition comprising at least one sunscreen agent, at least one water resistance agent, and at least one organic water insoluble thermoplastic polymer, wherein
- the water resistance agent is hydrolysed jojoba ester, and
the organic water insoluble thermoplastic polymer is ethylcellulose.

2. The sunscreen composition according to claim 1, wherein the water resistance agent is hydrolysed jojoba ester, and wherein the amount of hydrolysed jojoba ester in the composition is 0,1-1%.

3. The sunscreen composition according to claims 1-2, wherein the organic water insoluble thermoplastic polymer is ethylcellulose.

4. The sunscreen composition according to claims 1-3, wherein the organic water insoluble thermoplastic polymer is ethylcellulose, and wherein the amount of ethylcellulose in the composition is 0,01-0,5%.

5. The sunscreen composition according to claims 1-4, wherein the at least one sunscreen active is selected from the group consisting of Cinoxate, Polysilicone-15, Dioxybenzone, Homosalate, Menthyl anthranilate, Octocrylene, Padimate O, Phenylbenzimidazole sulfonic acid, Titanium dioxide, Trolamine salicylate, Zinc oxide, Benzophenone-3 , Ethylhexyl Methoxycinnamate, Octocrylene, Butyl Methoxydibenzoylmethane (BMBM), Diethylamino hydroxybenzoyl Hexyl Benzoate, Diethylhexyl Butamido Triazone, PABA, camphor benzalkonium methosulfate, terephthalidene dicamphor sulfonic acid, benzylidene camphor sulfonic acid, polyacrylamidomethyl benzylidene camphor, PEG-25 PABA, isoamyl p-methoxycinnamate, ethylhexyl triazone, drometrizole trielloxane, 4-methylbenzylidene camphor, 3-benzylidene camphor, ethylhexyl salicylate, ethylhexyl dimethyl PABA, benzophenone-4, methylene bis-benztriazolyl tetramethylbutylphenol, disodium phenyl dibenzimidazole tetrasulfonate, bis-ethylhexyloxyphenol methoxyphenol triazine, methylene bisbenzotriazolyl tetramethylbutylphenol, bisethylhexyloxyphenol methoxyphenyl triazine and any combination thereof, or salts or derivatives of any one or more of these compounds, or any combination hereof.

6. The cosmetic composition according to claims 1-5, wherein cosmetic composition does not comprise microplastics, such as acrylates/C10-30 alkyl acrylate crosspolymer, acrylates/C12-22 alkyl methacrylate copolymer, carbomer, dimethicone, hydroxyethyl acrylate/sodium acryloyldimethyl taurate copolymer, triacontanyl PVP, VP/Eicosene copolymer, or e.g. VP/Hexadecene copolymer.

7. The cosmetic composition according to claims 1-6, wherein cosmetic composition has an SPF in the range of SPF15-SPF50+.

8. The cosmetic composition according to claims 1-7, wherein cosmetic composition is selected from the group consisting of a lotion, a cream, a spray, an emulsion, a serum, a stick and a gel.

9. The cosmetic composition according to claims 1-8, wherein cosmetic composition is lotion.

10. The cosmetic composition according to claims 1-9, wherein cosmetic composition does not contain synthetic polymer microparticles "SPMs".

11. The cosmetic composition according to claims 1-10, wherein the composition does not comprise microplastics selected from the group consisting of acrylates/C10-30 alkyl acrylate crosspolymer, acrylates/C12-22 alkyl methacrylate copolymer, carbomer, dimethicone, hydroxyethyl acrylate/sodium acryloyldimethyl taurate copolymer, triacontanyl PVP, VP/Eicosene copolymer, and VP/Hexadecene copolymer.
